# EUROPEAN PATENT APPLICATION

(11) **EP 2 443 999 A1**
(43) Date of publication of application: **25.04.2012**
(21) Application number: 08763145.3
(22) Date of filing: 30.05.2008
(51) Int. Cl.: A61B 5/107

(54) **UTERINE PROLAPSE DIAGNOSTIC INSTRUMENT**

(71) Applicant: Santos Bellas, Gabriel Antonio, Bogota N/A (CO)
(72) Inventor: Santos Bellas, Gabriel Antonio, Bogota N/A (CO)
(74) Representative: Lahidalga de Careaga, Jose Luis
(86) International application number: PCT/IB2008/052108
(87) International publication number: WO 2010/064080

(57) **Abstract**

This invention is related to means for the normal or abnormal movement measurement of the uterus. Particularly, it refers to a device permitting to quantify the prolapse of the uterus or the vaginal vault after that the patient has had a hysterectomy. Such device or prolapsometer embracesa set of two elements, where the internal element is composed by a color strip with a color centimeters scale that moves and slides within the semi-cylindrical and transparent external element also known as a centimeters scale as well as visual indicators or normality or insufficiency found after the introduction to said strip in the uterine space according the distance ran by it.

## Description

### Background Art

SHORT INVENTION BACKGROUNDS

In our previous patent called *'Instrument for the measurement and diagnosis of the uterine and*/*or vaginal vault falling and measurement method'* Venezuelan patent No. 51.162 dated October 10th 1988, the need of supplying a mean that permitting to have, immediately, a precise and precocious diagnosis as the case may be, about the ligament sufficiency of the uterine prolapse as well as of the vaginal vault was proposed. In said patent and in spite of the advantages that it presents and has in its conception and use; a set of three pieces as well as different variants of construction in regards said three pieces set were established. Such set was based in a 'injector-type' body which 'cylinder' sat in the genitals or the external lips and the 'piston'penetrated in the uterine space. It also had a 'sliding t-shirt' on the cylinder permuting to determine the measurement.

The practice demonstrated that it was necessary to make some adjustments, both for the comfort of the patients as well as for the easiness of its construction and operation, which are expressed in the following objectives:

An objective of this invention is to supply a simpler device, composed by a semi-cylindrical slim body of easy trajectory along the vaginal conduct.

Another objective of this invention is to supply a device that only requires the use of two pieces, simplifying its construction and reading.

Another objection of this invention is to provide a device composing by a sliding element with differentiated color within another transparent element permitting to visualize the internal element displacement.

Another objective of this invention is to provide centimeter and comparative scales in both pieces permitting to quantify in numbers and immediately the scope of an eventual abnormality that the patient may has.

### Description of Drawings

The attached illustrative figures show the characteristics of the device in its different indicator facets of normality or the detection of pathologies in which:

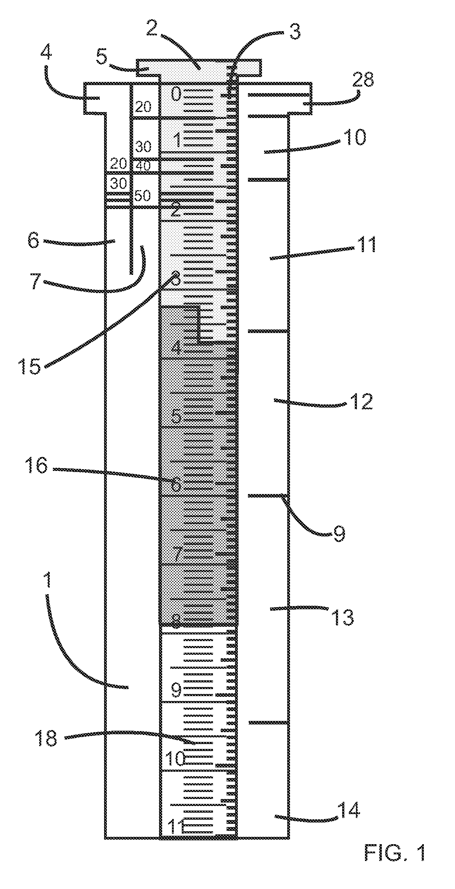

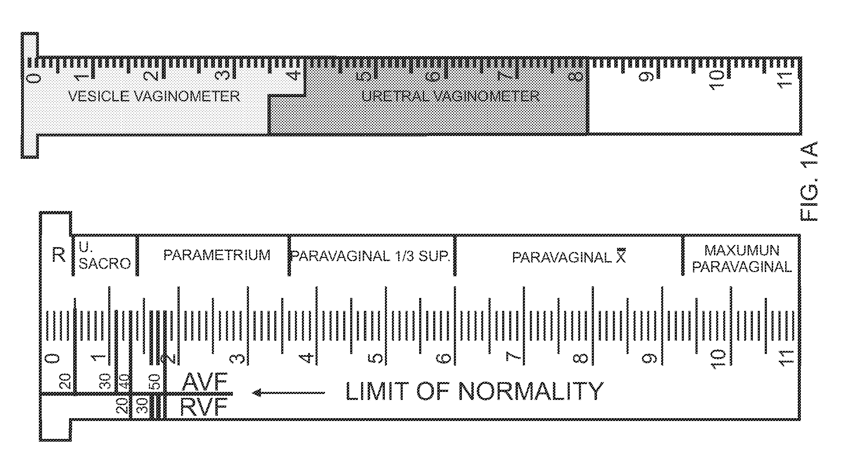

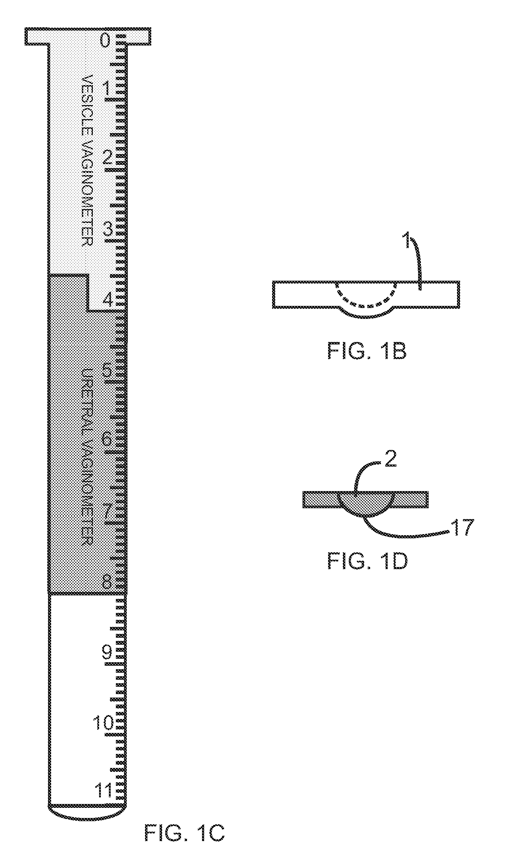

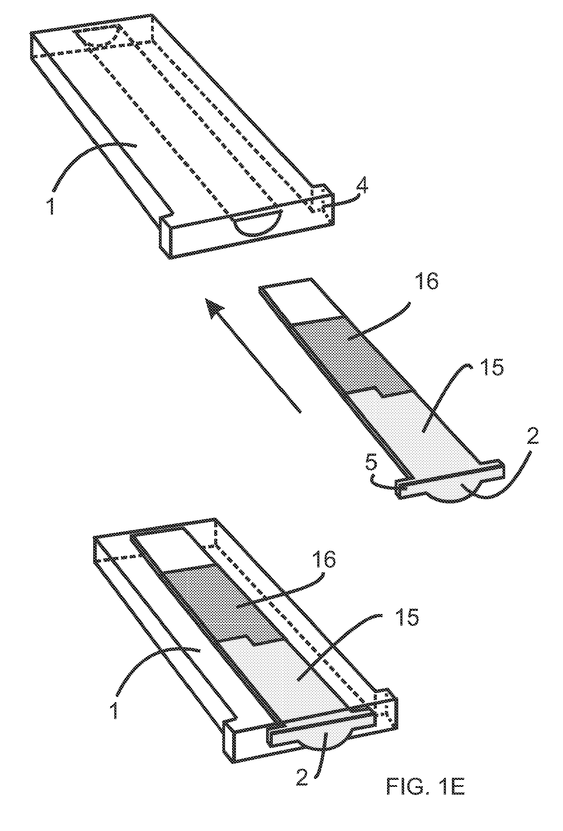

Figure 1 general in plant of the prolapsometer armed set where all the elements conforming it are shown.

Figure 1-a is a general view in the plant of the improved device of the invention showing the external fixed component and the internal mobile component with the corresponding scale and legends of indicative guideline at pathology level.

Figure 1-b is a lateral view of the left extremity of Figure 1-a where the semi-cylindrical are of such external body is observed.

Figure 1-c is a frontal view of the prolapsometer together with the set showing in the flat face the marking or millimeter measurements as well as the proper indicator legends required for the interpretation or normal or pathological diagnosis.

Figure 1-d is a view of the extreme of the internal mobile component where the semi-cylindrical are of such internal mobile component is observed.

Figure 1-e is a perspective of the prolapsometer.

Figure 2 shows a normal characteristic example of vaginometry.

Figures 3 and 4 show characteristic examples of results in the normal prolapsometries.

Figure 5, 6 and 7 show characteristic examples of results in pathological prolapsometries.

From the observation of the figures, it can be observed the structure of both bodies of the device, sliding each other where the major body or 'fixed' body (1) (semi-cylindrical external) hosts the 'mobile' body (2) (semi-cylindrical internal) in such a way that said mobile body (2) can run longitudinally and freely in body (1). Such body (1) is of transparent material and has marked a longitudinal scales (18) from 0 cm to 11 cm and in its left size an indicator legend of the buffer of the normal prolapsometry to effort with differentiated scales anteversoflexion - AVF (6) and scales retroversoflexion - RVF (7). Likewise, in both AVF (6) and RVF (7) sacles the buffers of normality are discriminated per age range. On the opposite size (9) or right size pathological, the non regular scales indicated with terms alluding the corresponding pathology such as lesion of Round ligaments (28), uterosacralligament (10), Parametrium ligament (11), paravaginal 1/3 and urine incontinence by effort (12), lesion of average paravaginal X (13) and maximun paravaginal (14); are defined according the appointed particular scale. Body (1) embracesalso extreme lateral projection (4) that acts as buffer with the patient's external genitals as will be explained after.

Mobile body (2) is essentially a mobile strip with a centimeters scale (3), also from zero to eleven, with two colors. The color (15) assigned to the vesicle vaginometer and the color (16) assigned to the uretral vaginometer, in such a way that it can be easily seen through the fixed body (1), that as aforesaid, is transparent. Such mobile body (2) also has lateral projections (5) for its introduction to the buffer within the vagina cavity, it is a flat strip in its anterior part with a semi-cylindricalscale (17) in its posterior part.

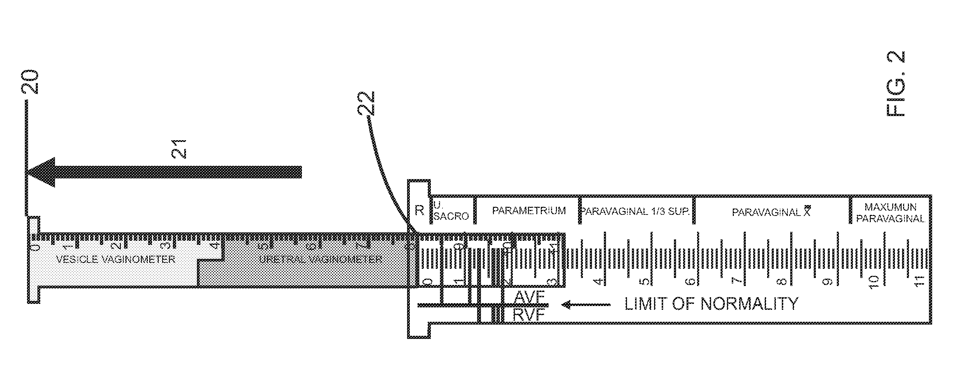

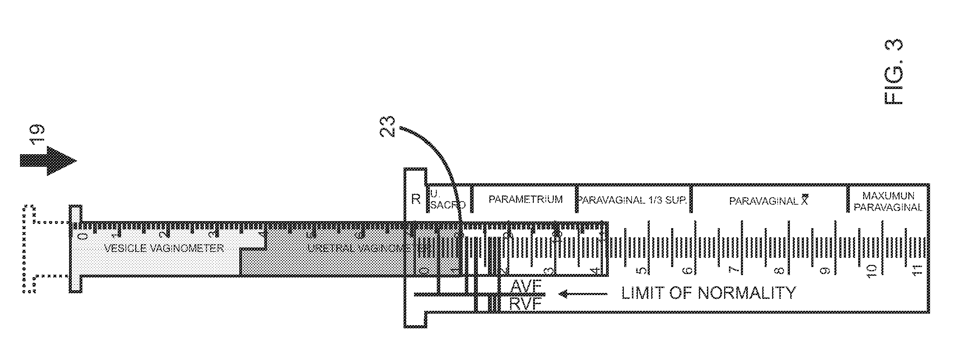

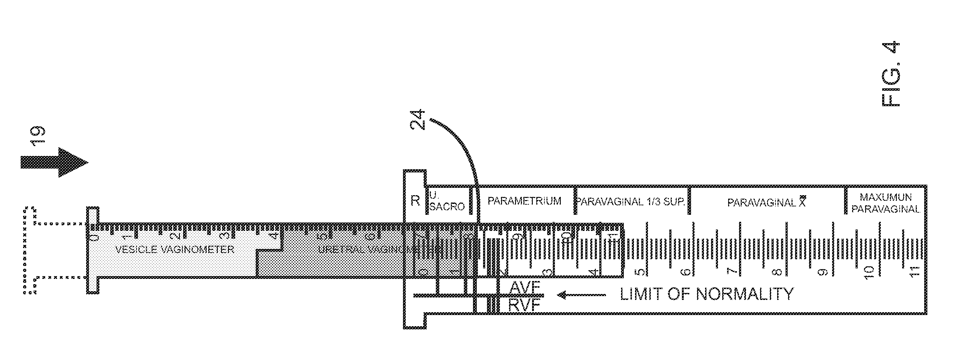

Figure 2 shows an example of measurement in the vaginal depth, representative of the device use, that will serve as guide for the remaining figures and the diagnosis derived from the different measurements. Placing a patient in the appropriate positions for the gynecological examination, a mobile strip 2 is introduced through the vagina until reaching the top (20) of the vaginal neck (ascendant lateral wide arrow) (21), taking the fixed body (1) to top (20) with the patient's external genitals. The reading reached, as shown in said figure, is a normal reading of 8cm (22) taken at '0' level of the fixed scale (18). In this stage (represented now for figures 3 and 4, uterus in AVF and RVF respectively) and to measure a possible prolapse, it is asked to the patient to make an abdominal wall contraction or effort in her stomach 'to struggle' that will result in a displacement (descendant lateral wide arrow) (19) of the mobile body (2) towards the inner of the fixed body (1). The reading is now measured by the left scale of the fixed (1) is of 1.8cm (23) and 2cm (24) respectively, representing such measurements at normal conditions, top values or figures for patients of 50 years and up.

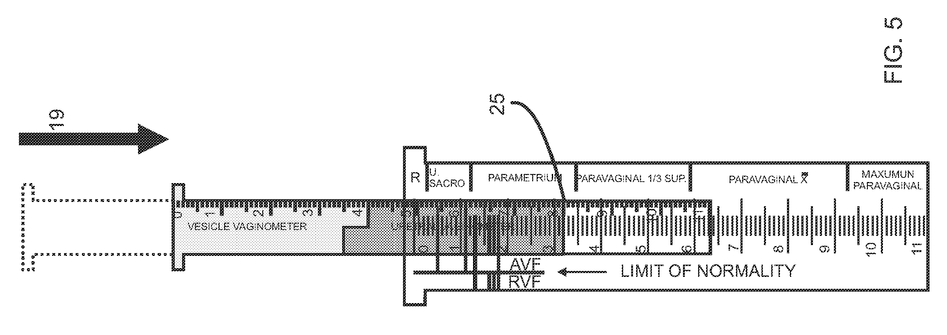

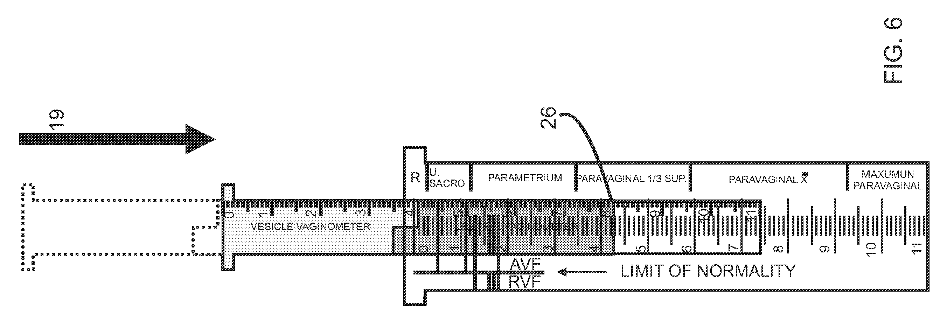

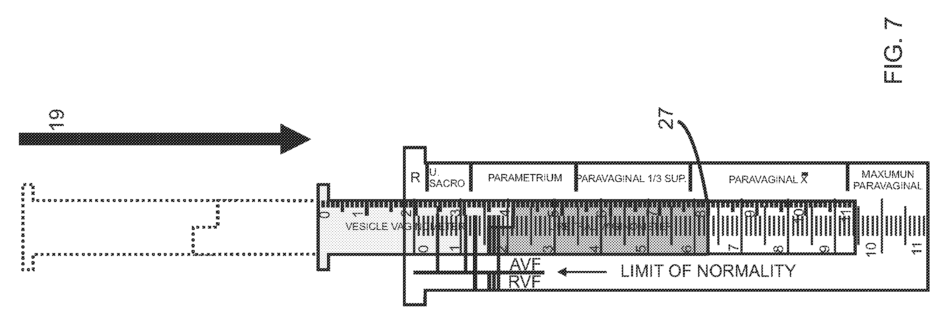

ObservingFigures 5, 6 and 7 are examples of pathological scale where in each one of them and after using the above technique with the device it is appreciateda successive increase in the displacement of the mobile body (2) towards the inner of the fixed body 1 which indicates by lateral wide arrow (19). In the case of figure 5 a reading of 3cm(25) in the prolapsometry, shows a lesion of the uterosacraland parametrium round ligaments. For the example of Figure 6 a reading of 4cm (26), the same above lesions plus the superior paravaginal 1/3, as well as stress urinary incontinence. Ultimately, for example of Figure 7, the reading 6cm (27) shows a lesion of average paravaginalX additionally.

The above lesions, as aforesaid, are indicated in the right lateral scale of the fixed body (1).

According with the above mentioned, the invention complies with the proposed objectives due to its simplicity and utility with which an immediate and safe vision of the pathological condition of a patient is reached, particularly after practicing a hysterectomy.

Also can be mentioned that the device can be built in a plastic and disposable material guarantying at the same time a total asepsis.

## Claims

1. An improved prolapsometer , designed to determine the conditions and scope in prolapse pathologies, that embracesa device composed by two bodies, one of them external fixed of flat anterior face later continued in a semi-cylindrical shape and the other one a internal mobile body also semi-cylindrical and floating the one within the other one, that embrace centimeter scales to indicate the movement of the mobile element as well as indicator scales of the type of ligaments lesions.

2. An improved prolapsometer according to assertion 1, **characterized** because its fixed body is made from a transparent material and the mobile body is made from a material colored in two colors in such a way that the scale and the displacement of the mobile element can be observed through the fixed body.

3. An improved prolapsometer according to assertion 1, where the path of the mobile body is measured through a short scale printed in the left size of the fixed body for a normal or healthy condition of the patient and the pathological conditions of a patient is measured through a scale printed in the right size of the fixed body with indicator legends that correspond to the insertion level of the mobile element and where the different types of affected ligaments is given by such level of insertion cumulatively.

4. An improved prolapsometer according to assertion 1 where both the fixed body as well as the mobile body have lateral projections in their operation extremities and where the ones of the fixed body seat in the patient's external genitals and the ones of the mobile body seat, after the introduction, in the top or the vaginal neck.
